(19) Europäisches Patentamt
European Patent Office
Office européen des brevets

(11) **EP 4 378 573 A1**

(12) **EUROPEAN PATENT APPLICATION**
published in accordance with Art. 153(4) EPC

(43) Date of publication:
**05.06.2024 Bulletin 2024/23**

(21) Application number: **22849851.5**

(22) Date of filing: **26.07.2022**

(51) International Patent Classification (IPC):
**B01F 33/30** (2022.01)   **B01F 33/3011** (2022.01)
**B01F 25/00** (2022.01)   **B01F 23/80** (2022.01)
**B01L 3/00** (2006.01)   **B01F 101/22** (2022.01)
**B82Y 5/00** (2011.01)

(52) Cooperative Patent Classification (CPC):
**B01F 23/80; B01F 25/00; B01F 33/30;**
**B01F 33/3011; B01F 2101/22; B01L 3/00;**
B82Y 5/00

(86) International application number:
**PCT/KR2022/010957**

(87) International publication number:
**WO 2023/008875 (02.02.2023 Gazette 2023/05)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **28.07.2021   KR 20210099471**
**25.07.2022   KR 20220091613**

(71) Applicant: **Inventage Lab. Inc.**
**Seongnam-si, Gyeonggi-do 13438 (KR)**

(72) Inventors:
• **LEE, Sanghun**
  **Seoul 07075 (KR)**
• **KIM, Ju Hee**
  **Seongnam-si Gyeonggi-do 13622 (KR)**
• **KIM, Dong Hoon**
  **Seongnam-si Gyeonggi-do 13530 (KR)**
• **CHON, Chan Hee**
  **Seongnam-si Gyeonggi-do 13165 (KR)**

(74) Representative: **Gulde & Partner**
**Patent- und Rechtsanwaltskanzlei mbB**
**Wallstraße 58/59**
**10179 Berlin (DE)**

(54) **CHIP FOR PREPARING LIPID NANOPARTICLES, SYSTEM FOR PREPARING LIPID NANOPARTICLES COMPRISING SAME, AND METHOD FOR PREPARING LIPID NANOPARTICLES**

(57)    The lipid nanoparticles manufacturing chip comprises a first raw material supply flow path, a second raw material supply flow path, and a mixer portion connected to the first raw material supply flow path and the second raw material supply flow path, and mixing a first raw material supplied through the first raw material supply flow path and a second raw material supplied through the second raw material supply flow path. The mixer portion comprises a first stabilizing unit, and a first mixing unit connected to the first stabilizing unit and mixing the first raw material and the second raw material with each other. Mixing of the first raw material and the second raw material is performed more in the first mixing unit than in the first stabilizing unit.

[FIG. 1]

**Description**

[TECHNICAL FIELD]

The present invention relates to a nano lipid particles manufacturing chip, a nano lipid particles manufacturing system comprising the nano lipid particles manufacturing chip, and a nano lipid particles manufacturing method, and more particularly, it relates to a nano lipid particles manufacturing chip for manufacturing nano lipid particles containing active ingredients such as mRNA, a nano lipid particles manufacturing system having the nano lipid particles manufacturing chip, and a nano lipid particles manufacturing method containing the active ingredient.

[BACKGROUND ART]

mRNA (messenger RNA) is a material at the stage before protein synthesis and contains genetic information. mRNA is accessible to a variety of therapeutic agents and can be used as a preventive or therapeutic vaccine, and missing proteins can also be synthesized through mRNA. The advantage of mRNA therapeutics compared to DNA is that they do not need to be delivered to the nucleus and are not inserted into the genome, so they do not cause permanent genetic diseases and are therefore highly safe. In addition, it is possible to synthesize missing proteins inside cells that cannot be accessed by protein therapeutics into mRNA. mRNA can have various sizes depending on the protein it expresses, and exists as a single strand. mRNA made from DNA escapes from the nucleus into the cytoplasm and meets live chromosomes to produce proteins. mRNA is in the spotlight as a next-generation gene therapy, but because it is single-stranded, it is known to have very low stability, so it is quickly decomposed by nucleic acid decomposition enzymes in the blood, it is known that and not only is it quickly excreted out of the body through the kidneys, but it also has a strong negative charge and does not easily pass through cell membranes.

In treatment using anionic drugs, including nucleic acids, safe and efficient drug delivery technologies have been studied for a long time, and various carriers and delivery technologies have been developed. A variety of research is being conducted on mRNA vaccines using a method of delivering mRNA by encapsulating it in nano lipid particles, and research on mass manufacturing systems for this is ongoing.

Recently, the method of delivering mRNA by encapsulating it in nano lipid particles has been receiving attention in fields such as pharmaceuticals, vaccines, and DDS (drug delivery system), but there are difficulties in mass production. There is a bulk manufacturing method that generates turbulence in a large container to mix, dilute, and concentrate, but it is difficult to obtain uniform quality of the manufactured nano lipid particles and difficult to control the manufacturing process.

Meanwhile, in an effort to solve this problem, methods for manufacturing nanolipid particles using a chip-type mixer are being studied, but due to difficulties in analyzing the characteristics of fluid flow, there are difficulties in producing more efficient and high-quality nanolipid particles.

[PRIOR ART LITERATURE]

[Patent literature]

US 10,835,878

[DISCLOSURE]

[Technical Problem]

Accordingly, the technical problem of the present invention has been conceived in this respect, and an object of the present invention is to provide a nano lipid particles manufacturing chip for producing nano lipid particles.

Another object of the present invention is to provide a nano lipid particles manufacturing system comprising the nano lipid particles manufacturing chip.

Another object of the present invention is to provide a nano lipid particles manufacturing method.

[Technical Solution]

According to an exemplary embodiment of the inventive concept, nano lipid particles manufacturing chip comprises a first raw material supply flow path, a second raw material supply flow path, and a mixer portion connected to the first raw material supply flow path and the second raw material supply flow path and mixing a first raw material supplied through the first raw material supply flow path and a second raw material supplied through the second raw material supply passage. The mixer portion comprises a first stabilizing unit, and a first mixing unit connected to the first stabilizing unit and mixing the first raw material and the second raw material with each other. Mixing of the first raw material and the second raw material is performed more in the first mixing unit than in the first stabilizing unit.

In an embodiment of the present invention, the first stabilizing unit comprises a left stabilizing flow path having a first width and a first length and a right stabilizing flow path having the first width and the first length.

In an embodiment of the present invention, the left stabilizing flow path and the right stabilizing flow path of the first stabilizing unit are symmetrical to each other and form an oval or circular shape.

In an embodiment of the present invention, the first mixing unit comprises a left mixing flow path having a second width and a right mixing flow path having a third width, and the second width and the third width are different from each other.

In an embodiment of the present invention, the left mixing flow path and the right mixing flow path of the first mixing unit form an oval or circular shape.

In an embodiment of the present invention, the first stabilizing unit is connected to the first raw material supply flow path and the second raw material supply flow path. The first raw material sequentially flows through the first raw material supply flow path, the first stabilizing unit, and the first mixing unit, and the second raw material sequentially flows through the second raw material supply flow path, the first stabilizing unit, and the first mixing unit, and mixing of the first raw material and the second raw material occurs at the interface between the first raw material and the first raw material, but more mixing of the first raw material and the second raw material occurs in the first mixing unit than in the first stabilizing unit.

In an embodiment of the present invention, the first mixing unit is connected to the first raw material supply flow path and the second raw material supply flow path, and the first raw material sequentially flows through the first raw material supply flow path, the first mixing unit, and the first stabilizing unit, and the second raw material sequentially flows through the second raw material supply flow path, the first mixing unit, and the first stabilizing unit.

In an embodiment of the present invention, the mixer portion further comprises a second stabilizing unit connected to the first mixing unit, and a second mixing unit connected to the second stabilizing unit.

According to an exemplary embodiment of the inventive concept, a nano lipid particles manufacturing system comprises a first raw material supply unit that supplies a first raw material, a second raw material supply unit that supplies a second raw material, a nano lipid particles manufacturing chip comprising a mixer portion that mixes the first raw material and the second raw material to form a mixed solution, and a lipid nanoparticle obtaining unit to obtain lipid nanoparticles manufactured from the nano lipid particles manufacturing chip. The mixer portion of the nano lipid particles manufacturing chip comprises a first stabilizing unit; and a first mixing unit connected to the first stabilizing unit and mixing the first raw material and the second raw material with each other.

In an embodiment of the present invention, the first stabilizing unit comprises a left stabilizing flow path having a first width and a first length and a right stabilizing flow path having the first width and the first length. The first mixing unit comprises a left mixing flow path having a second width and a right mixing flow path having a third width, and the second width and the third width are different from each other.

According to an exemplary embodiment of the inventive concept, a nano lipid particles manufacturing method comprises a step of preparing a first raw material containing an active ingredient and a second raw material containing lipids, a step of manufacturing a nano lipid particles, and a step of post-processing of manufacturing a final product by filtering and filling the solution containing the nano lipid particles into individual containers. The step of manufacturing a nano lipid particles is performed on a lipid nanoparticles manufacturing chip with a flow path formed, and comprises a stabilizing step in which the first raw material and the second raw material pass through a stabilizing unit including a left stabilizing flow path and a right stabilizing flow path having the same width and length, and a mixing step in which the first raw material and the second raw material are mixed with each other while passing through a mixing unit including a left mixing flow path and a right mixing flow path having different widths.

In an embodiment of the present invention, the left stabilizing flow path and the right stabilizing flow path of the stabilizing unit are symmetrical to each other and form an oval or circular shape. The left mixing flow path and the right mixing flow path of the mixing unit form an oval or circular shape.

In an embodiment of the present invention, the stabilizing step and the mixing step are alternately repeated at least twice.

[Advantageous Effects]

According to the exemplary embodiments of the present invention, a mixer portion of the nano lipid particles manufacturing chip comprises a stabilizing unit and a mixing unit that are arranged alternately. According to the flow path design of the stabilizing unit and the mixing unit, high-quality nanolipid particles can be manufactured more stably and efficiently.

However, the effects of the present invention are not limited to the above effects and may be variously expanded without departing from the spirit and scope of the present invention.

[Description of Drawings]

FIG. 1 is a schematic diagram of a nano lipid particles manufacturing system according to an embodiment of the present invention.

FIG. 2 is a detailed view illustrating the mixer portion of the nano lipid particles manufacturing chip of the nano lipid particles manufacturing system of FIG. 1.

FIG. 3 is a view for explaining the fluid flow in the mixer portion of FIG. 2.

FIG. 4 is a detailed view illustrating the mixer portion of the nano lipid particles manufacturing chip of the nano lipid particles manufacturing system according to an embodiment of the present invention.

FIG. 5 is a view illustrating the nano lipid particles manufacturing chip of the nano lipid particles manufacturing system according to an embodiment of the present invention.

FIG. 6 is a view illustrating simulation and experiment results of the fluid flow of the nano lipid particles manufacturing chip of the nano lipid particles manufacturing system according to an embodiment of the present invention.

FIG. 7 is a view for explaining mixing efficiency (Mixing Index) calculation for the experiment of FIG. 6.

FIG. 8 is a graph illustrating simulation and experiment results of the degree of mixing according to the length of the mixer portion of the nano lipid particles manufacturing chip of the nano lipid particles manufacturing system according to an embodiment of the present invention.

FIG. 9 is a flow chart illustrating a nano lipid particles manufacturing method according to an embodiment of the present invention.

FIG. 10 is a detailed flowchart illustrating the steps for manufacturing nano lipid particles on a chip in the lipid nanoparticle manufacturing method of FIG. 9.

[Mode for Invention]

Hereinafter, exemplary embodiments of the present invention will be described in more detail with reference to the drawings.

Since the present invention may have various changes and have various forms, specific exemplary embodiments are illustrated in the drawings and described in detail in the text. However, it is not intended to limit the present invention to the specific disclosed form, and it will be appreciated that the present invention includes all modifications, equivalences, or substitutions included in the spirit and the technical scope of the present invention.

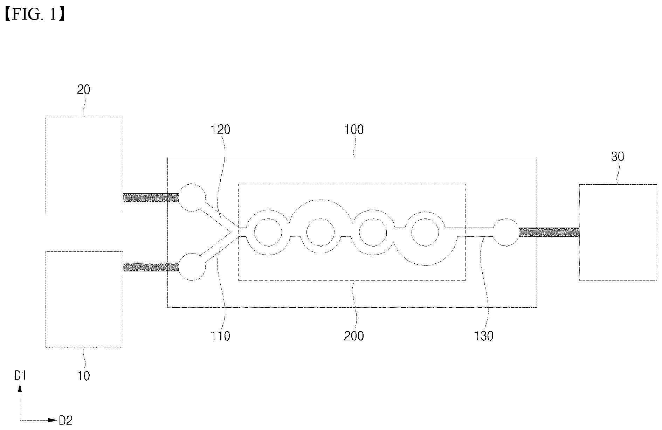

FIG. 1 is a schematic diagram of a nano lipid particles manufacturing system according to an embodiment of the present invention.

Referring to FIG. 1, the lipid nanoparticle manufacturing system comprises a first raw material supply unit 10 containing mRNA, a second raw material supply unit 20 containing lipids, a lipid nanoparticle obtaining unit 30, and a nano lipid particles manufacturing chip 100.

The first raw material supply unit 10 stores a first raw material and provides the first raw material to the nano lipid particles manufacturing chip 100. The first raw material may be a solution containing mRNA. For example, the first raw material may include mRNA and water, but is not limited thereto. For example, the first raw material may include various types of active ingredients such as mRNA as well as nucleic acids such as miRNA, siRNA, and DNA, or CRISPR.

The second raw material supply unit 20 stores a second raw material and provides the second raw material to the nano lipid particles manufacturing chip 100. The second raw material may be a solution containing lipid. For example, the second raw material may include lipid and ethanol.

The lipid nanoparticle manufacturing chip 100 may produce a lipid nanoparticle solution containing mRNA by mixing the first raw material and the second raw material.

The lipid nanoparticle manufacturing chip 100 may include a first raw material supply flow path 110, a second raw material supply flow path 120, a mixer portion 200, and a lipid nanoparticle obtaining flow path 130.

The first raw material supply flow path 110 may receive the first raw material from the first raw material supply unit 10 and deliver it to the mixer portion 200. The second raw material supply flow path 120 may receive the second raw material from the second raw material supply unit 20 and deliver it to the mixer portion 200.

The mixer portion 200 is connected to the first and second raw material supply flow paths 110 and 120, and may mix the first raw material and the second raw material to prepare a mixed solution containing nano lipid particles. The mixer portion 200 may be a microfluidic mixer commonly used in microchannels, such as a chaotic mixer or a herringbone mixer. At this time, through mixing of the first raw material and the second raw material within the flow path, a mixed solution containing nano lipid particles formed by self-assembly of lipids and mRNA at the interface of the two fluids may be prepared.

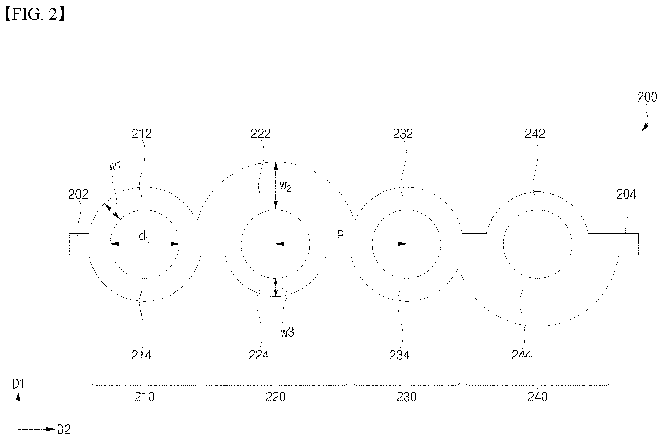

The mixer portion 200 may include at least one pair of stabilizing units and mixing units arranged alternately. In order for the active ingredient of the first raw material to be surrounded by the lipid of the second raw material to form high-quality nano lipid particles containing the active ingredient, not only mixing between solutions (First raw material and second raw material) but also the area of the contact surface between solutions, form, and flow of surrounding solution, etc. all have an effect. An optimal design for this is required, and the embodiment of the present invention achieves this by configuring the mixer portion 200 to include at least one pair of stabilizing units and mixing units as described above. The mixer portion 200 will be described in detail later in the description of FIG. 2.

The lipid nanoparticle obtaining flow path 130 may be connected to the mixer portion 200. The lipid nanoparticle obtaining unit 30 may obtain a mixed solution containing the nano lipid particles formed in the mixer portion through the lipid nanoparticle obtaining flow path 130.

Thereafter, the mixed solution is diluted or concentrated as necessary to form a lipid nanoparticle solution of the desired concentration, and then the solution containing the nano lipid particles is filtered and filled into individual containers to prepare the final product.

The nano lipid particles manufacturing chip 100 may have various structures to form a flow path therein. For example, the nano lipid particles manufacturing chip 100 may have a structure in which a groove includes a first substrate and a second substrate bonded to the first substrate on the first substrate.

FIG. 2 is a detailed view illustrating the mixer portion of the nano lipid particles manufacturing chip of the nano lipid particles manufacturing system of FIG. 1.

Referring to FIG. 2, the mixer portion 200 may comprise a merging unit 202, a first stabilizing unit 210, a first mixing unit 220, a second stabilizing unit 230, a second mixing unit 240, and a discharge unit 204.

The merging unit 202 is a point where a first raw material supply flow path(1 10 in FIG. 1) and a second raw material supply flow path(120 in FIG. 1) are merged. The first stabilizing unit 210 may be connected to the merging unit 202.

The first stabilizing unit 210 may include a left stabilizing flow path 212 having a first width w1 and having a first length, and a right stabilizing flow path 214 having the first width w1 and having the first length. Accordingly, the left stabilizing flow path 212 and the right stabilizing flow path 214 of the first stabilizing unit 220 may be symmetrically oval or circular with respect to a second direction D2. The first raw material and the second raw material begin to mix in the merging unit 202, and may pass through the flow path of the first stabilizing unit 220 to ensure that nano lipid particles formed by self-assembly at the mixing interface have sufficient structure formation time.

The first mixing unit 220 is connected to the first stabilizing unit 210. The first mixing unit 220 may include a left mixing flow path 222 having a second width w2 and a right mixing flow path 224 having a third width w3. The second width w2 and the third width w3 are different from each other. Accordingly, the left mixing flow path 222 and the right mixing flow path 224 of the first mixing unit 220 may have an asymmetric oval or circular shape. More mixing of the first raw material and the second raw material may occur in the first mixing unit 220 than in the first stabilizing unit 210.

The second stabilizing unit 230 is connected to the first mixing unit 220. The second stabilizing unit 230 may include a left stabilizing flow path 232 having the first width w1 and the first length, and a right stabilizing flow path 234 having the first width w1 and the first length. Accordingly, the left stabilizing flow path 232 and the right stabilizing flow path 234 of the second stabilizing unit 230 may be symmetrically oval or circular. Nano lipid particles formed by self-assembly at the fluid mixing interface in the first mixing unit 220 may pass through the flow path of the second stabilizing unit 230 so that they have sufficient structure formation time.

The second mixing unit 240 is connected to the second stabilizing unit 230. The second mixing unit 240 may include a left mixing flow path 242 having the third width w3 and a right mixing flow path 244 having the second width w2. Accordingly, the left mixing flow path 242 and the right mixing flow path 244 of the second mixing unit 240 may have an asymmetric oval or circular shape, and may be symmetrical to the shape of the first mixing unit 220. The first raw material and the second raw material may be mixed more in the second mixing unit 240 than in the second stabilizing unit 230.

The discharge unit 204 may be connected to the second mixing unit 240 and may be connected to the lipid nanoparticle obtaining flow path (130 in FIG. 1).

In this embodiment, the mixer portion 200 includes two pairs of stabilizing units and mixing units arranged alternately, but is not limited to this. For example, various structures are possible, such as a greater number of stabilizing units and mixing units being arranged alternately with each other, or a mixing unit being placed before the stabilizing unit.

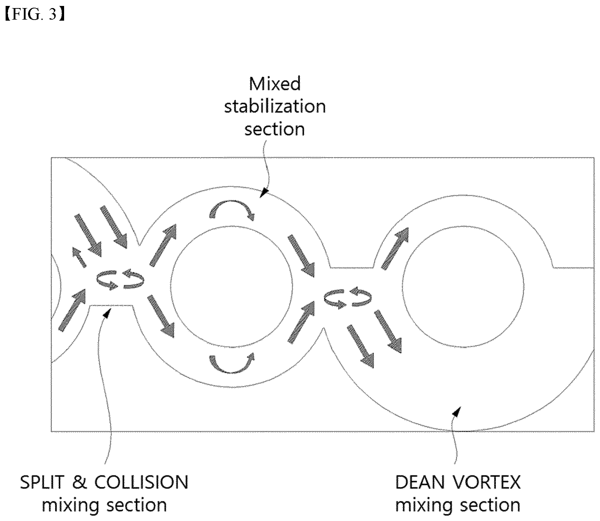

FIG. 3 is a view for explaining the fluid flow in the mixer portion of FIG. 2.

Referring to FIG. 3, in general, nano lipid particles are generated through the self-assembly process occurring at the interfacial area between two different solutions (water-based and oil-based) when mixed. When designing a nano lipid particles manufacturing chip using microfluidic technology, key factors include a) the flow conditions of the applied solutions, b) the Reynolds number, which quantifies the dynamics relationship of the relative force due to the inertial and viscosity of the internal solution as a dimensionless number, and c) analysis of diffusion phenomena arising from differences in concentration at the interfacial area and momentum generated by the flow of solution fluids.

In addition, the flow rate conditions of Flow Rate Ratio (FRR) and Total Flow Rate (TFR), which are the supply ratio of water base and oil base solutions, may be considered. FRR is the ratio of each of the two solutions and is defined as $\frac{Flow\ Rate\ of\ Water\ Base\ Solution}{Flow\ Rate\ of\ Oil\ Base\ Solution}$, and the sum of the flow rates of the two solutions is defined as TFR. Depending on the values of FRR and TFR, the degree of contact between the two solutions and the difference in concentration at the contact surface change, so they should be considered in the design of the nano lipid particles manufacturing chip.

Through the dimensionless Reynolds number, it is possible to predict the mixing of the solution and the occurrence of interfacial area inside the channel (flow path) of the nano lipid particles manufacturing chip. The method of deriving the Reynolds number (Re) from the flow inside the channel is as shown in <Equation 1> below.

$$Re = \frac{Ineirial\ Force}{Viscous\ Force} = \frac{\rho U_{mean} D_h}{\mu}$$

<Equation 1> (Reynold Number calculation formula inside the channel)

$\rho$ is the density of the internal solution, $U_{mean}$ is the average velocity of the solution, $D_h$ is the hydraulic diameter reflecting the characteristics of the diameter inside the channel, and $\mu$ is the viscosity coefficient of the solution. Typically, when the Reynolds number is less than 2,300, the flow of the solution can be defined as laminar flow, and when the Reynolds number is higher than 2,300, it goes through transition flow and finally forms turbulent flow. As can be seen from the above variables, by appropriately adjusting and tuning the dimensionless Reynolds number, which represents the ratio of inertia and viscosity, through the degree of advection of the internal solution and diffusion and momentum diffusion due to the concentration difference at the contact surface, it has a positive effect on the self-assembly of nanolipid particles inside the solution, making it possible to efficiently manufacture high-quality nanolipid particles. According to the above review, the channel of the nano lipid particles manufacturing chip was designed by considering the relationship between the degree of advection based on the Reynolds number of flow conditions, the influence of momentum diffusion, and the influence of diffusion due to concentration difference.

Meanwhile, the hydraulic diameter, which reflects the characteristics of the channel inner diameter, has an important influence on the adjustment and tuning of the Reynolds number, and the hydraulic diameter can be calculated using <Equation 2> below, assuming that the inside of the channel is full of solution.

$$D_h = \frac{4 \times Channel\ Cross\ Section\ Area}{Fluid\ Wetted\ Perimeter}$$

$$D_h = \frac{4 \times \frac{\pi}{4} D^2}{\pi D} = D\ (Circular\ channel\ cross\ section, diameter\ D)$$

$$D_h = \frac{4 \times a^2}{4a} = a\ (Square\ channel\ cross\ section, side\ a)$$

$$D_h = \frac{4ab}{2a + b}\ (Rectangular\ channel\ cross\ section, base\ a, height\ b)$$

<Equation 2> (Hydraulic diameter calculation formula according to the internal cross-sectional area of the channel)

In addition, Reynolds number is deeply related to the properties of internal solutions and fluids. In particular, density and viscosity are factors to be considered. The degree of mixing varies depending on the FRR, but basically, it can be viewed as a mixed solution of Water Base and Oil Base, and water and oil have poor miscibility between solutions, so there is a big difference in the viscosity of the mixed solution depending on the mixing ratio and FRR. When using ethanol and water, which are widely used in the production of nanolipid particles, the change in viscosity depending on the degree of mixing of water and ethanol can be referred to the study by Khattab, I, as shown in <Table. 1> below.

<Table. 1> (Density ($\rho$), viscosity ($\mu$), surface tension ($\sigma$) according to the ethanol mole fraction ($x_2$) of the mixed solution, and change measured values (Exp) and calculated values (Cal) of Molar Volume (V))

| $x_2$ | $\rho \times 10^{-3}$ (kg·m$^{-3}$) | | $\eta \times 10^3$ (Pa·s) | | $\sigma \times 10^3$ (N·m$^{-1}$) | | $V \times 10^6$ (m$^3$ mol$^{-1}$) | |
|---|---|---|---|---|---|---|---|---|
| | Exp | Cal | Exp | Cal | Exp | Cal | Exp | Cal |
| | | | | | T=298 (K) | | | |
| 0.000 | 0.9971 | 0.9971 | 0.8914 | 0.8914 | 72.15 | 72.15 | 18.05 | 18.05 |
| 0.033 | 0.9823 | 0.9855 | 1.1703 | 1.1441 | 51.44 | 55.82 | 19.27 | 19.21 |
| 0.072 | 0.9709 | 0.9723 | 1.5963 | 1.4346 | 42.70 | 44.12 | 20.61 | 20.59 |
| 0.117 | 0.9577 | 0.9575 | 2.0140 | 1.7371 | 36.87 | 35.99 | 22.22 | 22.25 |
| 0.171 | 0.9419 | 0.9408 | 2.2875 | 2.0019 | 33.43 | 30.67 | 24.19 | 24.28 |
| 0.236 | 0.9232 | 0.9220 | 2.3869 | 2.1609 | 30.11 | 27.62 | 26.66 | 26.78 |
| 0.316 | 0.9021 | 0.9011 | 2.4236 | 2.1540 | 28.76 | 26.42 | 29.80 | 29.94 |
| 0.419 | 0.8788 | 0.8782 | 2.2249 | 1.9796 | 27.35 | 26.47 | 33.85 | 34.00 |
| 0.552 | 0.8529 | 0.8536 | 2.0271 | 1.7293 | 25.78 | 26.19 | 39.28 | 39.37 |
| 0.735 | 0.8266 | 0.8266 | 1.6594 | 1.5368 | 24.12 | 22.89 | 46.74 | 46.84 |
| 1.000 | 0.7858 | 0.7858 | 1.0995 | 1.0995 | 22.07 | 22.07 | 58.63 | 58.63 |

In the case of water without ethanol mixed at room temperature (298K), viscosity ($\mu$) has a calculated value of 0.8914 *mPa · s* and a measured value of 0.8914 *mPa · s,* and in the case of pure ethanol, it has a calculated value of 1.099 *mPa · s* and a measured value of 1.099 *mPa · s*. However, due to the low miscibility of water and ethanol, the viscosity value is 0.316 for ethanol mole fraction due to nano-sized particles generated during mixing, the calculated value is 2.161 mPa·s, and the measured value is 2.423 mPa·s, showing a significant difference. Therefore, it is possible to design an optimized channel of a nano lipid particles manufacturing chip only by carefully analyzing the degree of FRR and the resulting viscosity change and calculating the Reynolds number more accurately.

Meanwhile, the diffusion phenomenon that occurs at the contact surface between two solutions can be divided into two cases and analyzed. First, diffusion from a microscopic perspective due to concentration differences between solutions, and diffusion from a macroscopic momentum perspective due to changes in viscosity and fluid properties of the mixed solution that occur during mixing.

In the case of diffusion due to concentration difference, the relationship between the diffusion amount of the substance diffusing through Fick's Law and the concentration difference can be quantified through several assumptions and reflected in the design, as shown in <Equation. 3> below.

$$J_x = -D \frac{\partial n}{\partial x}$$

<Equation. 3> (Fick's Law, diffusion analysis law due to concentration difference, $J_x$ is

the diffusion flow rate in the particle x-axis direction, D is the diffusion constant, and n is the

particle concentration)

Analysis from the perspective of momentum will lead to a derivation method using the two models most commonly used in fluid dynamics analysis, the mass conservation equation and Navier-Stoke's equation, using the correlation between flow rates. (Refer to <Equation 4> below).

$$\nabla \cdot u = 0$$

$$\frac{\partial u}{\partial t} + u \cdot \nabla u + \frac{1}{\rho} p = g + \mu \nabla \cdot \nabla u$$

<Equation. 4> (Mass continuity equation (above) and Navier-Stokes equation (below),

$\nabla$ is the Dell operator, u is the flow rate, t is time, p is pressure)

Using these two methods, the performance of the channel design of various nano-lipid particle manufacturing chips was analyzed through simulation and fluid dynamics analysis, and verified through experiment and comparison with simulation and analysis values.

In embodiments of the present invention, the flow of fluid is divided into a phenomenon of transferring particles or substances in the main flow direction of the fluid through advection using laminar flow. And the channel design was designed based on the operating principle of chaotic advection, which adds a crushing chaos effect. The chaotic advection method can overcome the biggest disadvantage of the existing advection phenomenon, which is that transport is limited to the unidirectional nature of the main fluid flow, and can achieve higher efficiency mixing between solutions.

In order to enhance the passive chaotic advection effect due to differences in geometric structures in microfluidic-based chips, high mixing efficiency can be achieved even with low pressure changes by a) Dean Vortex through the internal channel of the chip in the form of a circular structure, and b) designing the channel in the form of splitting, rearranging, and recombining the fluid flow.

However, this is only a way to increase the mixing efficiency of two different solutions (water base, oil base), and cannot be fully applied to the manufacture of nano lipid particles, and it is necessary to further consider the characteristics of nano lipid particles manufacturing that self-assembles at the contact surface between solutions that occur during mixing.

The applicant designed a chip for nano lipid particles including a stabilizing unit and a mixing unit according to embodiments of the present invention by precisely tuning the channel design so that nano lipid particles can be produced with high efficiency by maximizing self-assembly at the contact surface of nano lipid particles manufacturing and to increase mixing efficiency. By applying a symmetrical and asymmetric structure in addition to the above-mentioned general properties using chaotic advection, it is designed and engineered to manufacture high-quality nano lipid particles more stably and efficiently as well as efficient mixing of solution fluids.

Referring to FIG. 3, it is designed to increase not only mixing but also self-assembly of nano lipid particles, by increasing the contact surface of the two solutions due to the effect of the Dean vortex as it passes through each channel through circular symmetric and asymmetric structures. In particular, the strength of the Dean vortex can be defined by the dimensionless number Dean Number, as shown in <Equation. 5> below.

$$\text{De} = \frac{\rho U\_mean \, D\_h}{\mu} \sqrt{(D\_h / [\![2R]\!]\_c)}$$

<Equation. 5> (Dean Number calculation formula inside circular structure channel)

Dean Number is as the relationship between inertial force, centrifugal force, and viscous force applied to the fluid in a circular channel, where $\rho$ is the density of the internal solution, U_mean is the average velocity of the solution, D_h is the hydraulic diameter, $\mu$ is the viscosity coefficient of the solution, and R_c is the radius of curvature. By specifying and adjusting the channel width of the asymmetric section and the ratio of the wide section (see w2 in Figure 2) and the narrow section (see w3 in Figure 2), this allowed the formation of an optimal contact surface between solutions for the production of nano lipid particles, by changing the hydraulic diameter and radius of curvature between asymmetric channels, a) the strength and influence of the Dean vortex in the asymmetric section are increased, and b) higher mixing efficiency is achieved in a specific section by increasing the section affected by the Dean vortex. Also, by applying a mixture of symmetrical and asymmetrical structures, the symmetrical and asymmetrical connection sections where the flows of the flow path meet again have increased and expanded, allowing for increased contact between solutions and highly efficient mixing through asymmetric inertial collisions in the flow path and the formation of detailed vortices in various directions.

In other words, in this embodiment, nano lipid particles can be formed and stabilized through a self-assembly process by splitting the fluid flow, colliding and mixing, and going through a mixing stabilization reaction section. And, the mixing and self-assembly process can proceed efficiently through Dean Vortex.

In addition, it was designed to achieve homogeneity mixing sequentially rather than immediate homogeneity mixing between water and oil base solutions through a serial arrangement of relatively low-efficiency mixing in the symmetric section and high-efficiency mixing in the asymmetric section. This is due to the concentration of a specific Oil Base solution, where initial lipid-based initial vesicles, which are produced by gathering in the form of disks due to a hydrophobic tail part when the phospholipid dissolved in the oil base solution meets the water base solution at the contact surface. For example, ethanol is formed more actively than in critical ethanol concentration, which is considered to be very closely affected by the residence time in the mixing process. Therefore, based on the concentration of the oil base solution, it is designed to perform sequential dilution rather than rapid dilution, which increases the efficiency of manufacturing nanolipid particles by increasing the retention time of the mixed solution at Critical Concentration.

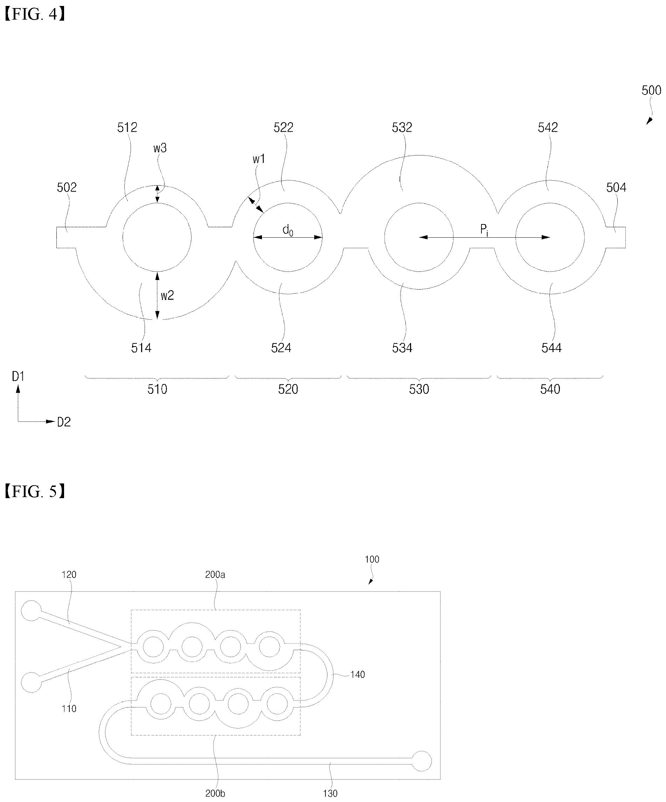

FIG. 4 is a detailed view illustrating the mixer portion of the nano lipid particles manufacturing chip of the nano lipid particles manufacturing system according to an embodiment of the present invention.

Referring to FIG. 4, a mixer portion 500 of the nano lipid particles manufacturing chip is substantially the same as the mixer portion 500 of the nano lipid particles manufacturing chip in FIG. 2, except for the arrangement of the stabilizing unit and the mixing unit. Therefore, repeated explanations are omitted.

The mixer portion 500 may comprise a merging unit 502, a first mixing unit 510, a first stabilizing unit 520, a second mixing unit 530, a second stabilizing unit 540, and a discharge unit.

The merging unit 502 is a point where a first raw material supply flow path and a second raw material supply flow path are merged. The first mixing unit 510 may be connected to the merging unit 502.

The first mixing unit 510 may include a left mixing flow path 512 having a third width w3 and a right mixing flow path 514 having a second width w2. The second width w2 and the third width w3 are different from each other.

The first stabilizing unit 520 is connected to the first mixing unit 510. The first stabilizing unit 520 may include a left stabilizing flow path 522 having a first width w1 and a first length and a right stabilizing flow path 524 having the first width w1 and the first length.

The second mixing unit 530 is connected to the first stabilizing unit 520. The second mixing unit 530 may include a left mixing flow path 532 having the second width w2 and a right mixing flow path 534 having the third width w3.

The second stabilizing unit 540 is connected to the second mixing unit 530. The second stabilizing unit 540 may include a left stabilizing flow path 542 having the first width w1 and the first length and a right stabilizing flow path 544 having the first width w1 and the first length.

The discharge unit 204 is connected to the second stabilizing unit 540 and may be connected to a lipid nanoparticle obtaining flow path.

FIG. 5 is a view illustrating the nano lipid particles manufacturing chip of the nano lipid particles manufacturing system according to an embodiment of the present invention.

Referring to FIG. 5, a nano lipid particles manufacturing chip is substantially the same as a nano lipid particles manufacturing chip in FIG. 1, except that a mixer portion is divided into a first mixer portion 200a and a second mixer portion 200b, and a connection flow path 140 connecting them is further formed. Therefore, repeated explanations are omitted.

The lipid nanoparticle chip 100 may include a first raw material supply flow path 110, a second raw material supply flow path 120, a first mixer portion 200a, a connection flow path 140, a second mixer portion 200b, and a lipid nanoparticle obtaining flow path 130.

The first mixer portion 200a and the second mixer portion 200b are connected by the connection flow path 140. Each of the first mixer portion 200a and the second mixer portion 200b may include a stabilizing unit and a mixing unit arranged alternately. Even if the number of stabilizing units and mixing units increases, the spatial design of the lipid nanoparticle chip can be streamlined by arranging them in a plurality of rows or columns and connecting them using connection channels.

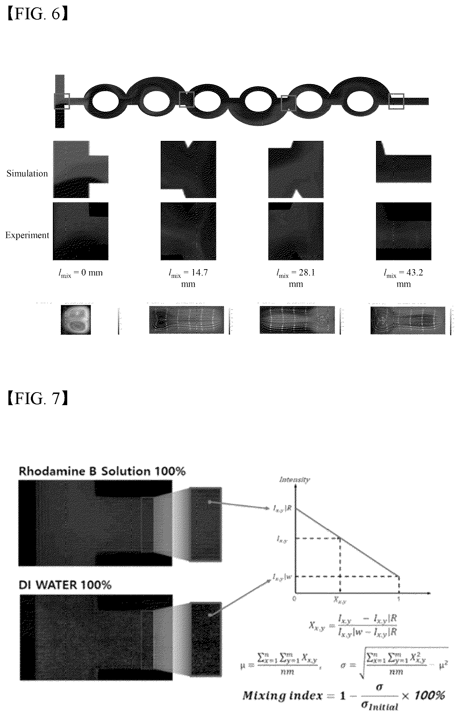

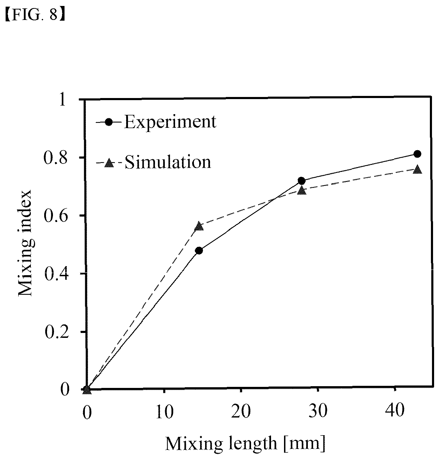

FIG. 6 is a view illustrating simulation and experiment results of the fluid flow of the nano lipid particles manufacturing chip of the nano lipid particles manufacturing system according to an embodiment of the present invention. FIG. 7 is a view for explaining mixing efficiency (Mixing Index) calculation for the experiment of FIG. 6. FIG. 8 is a graph illustrating simulation and experiment results of the degree of mixing according to the length of the mixer portion of the nano lipid particles manufacturing chip of the nano lipid particles manufacturing system according to an embodiment of the present invention.

Referring to FIG. 6 to FIG. 8, simulation values of flow in four locations (Refer to the box markings, 4 different mixing lengths (lmix)) on the lipid nanoparticles manufacturing chip, the actual corresponding experimental result value (experiment), and the values that simulate the fluid flow in the cross section of the channel (flow path) at the corresponding location are shown (bottom row).

The working fluid used in the experiment was deionized water as a water base solution, and was an oil base solution, which was a mixture of ethanol and Rhodamine B diluted to a concentration of 50 $\mu$M. The photographing of the contact surface between the solutions inside the mixer portion of the two solutions was taken in a direction perpendicular to the upper surface of the mixer portion. Intensity based on the pixels of the captured image was analyzed in each image through n-by-m pixel by dividing the horizontal direction (x-axis) of the image into n numbers and the vertical direction (y-axis) into m numbers. Photographs were taken when the fluid flow appropriate for each flow rate at the location to be measured stabilized in a steady state that does not change over time. Additionally, for more accurate relative analysis, images were taken when a mixed solution of Rhodamine B and ethanol was flowing and when only deionized water was flowing. When only the mixed solution of Rhodamine B and ethanol flows, it is reflected as a value of 100% mixing, that is, full mixing, and when only deionized water flows, it is reflected as a value of 0% mixing, a state where mixing has not yet progressed. After converting the images taken in the experiment to grayscale, the pixel intensity of each pixel is calculated. The standard deviation ($\sigma$) is obtained through linear interpolation between Pixel Intensity and Intensity values at 100% Mixing and 0% Mixing in each photographed image. Inside the micromixer, the mixing efficiency (Mixing Index) was calculated and quantified through the ratio of the standard deviation ($\sigma_{initial}$) in the initial section image where mixing has not yet occurred. (see Figure 7)

To verify the mixing efficiency (Mixing Index) with the experimental results, analysis was performed using COMSOL Multiphysics, one of the programs commonly used in numerical analysis. The flow field distribution, which is a coordinate function of space and time, of the solution flow inside the mixer portion when the flow rate is supplied was calculated by analyzing the Naibe-Stoke equation as in <Equation. 4> above. The conditions applied to calculate the flow field distribution reflected three dimensions (x-, y-, and z-direction), steady state in which the flow of the internal solution is not dependent on time, and incompressibility assuming that the density of the solution does not change at a constant rate, etc. The applied physical properties are the density of water ($\rho_{water}$) 997.1 [kg/m$^3$] and the viscosity coefficient ($\mu_{water}$) 0.891[$mPa{\cdot}s$]. In the case of ethanol, the density ($\rho_{ethanol}$) is 785.8 [kg/m$^3$] and the viscosity coefficient ($\mu_{ethanol}$) is 1.099[$mPa{\cdot}s$]. The density of the mixed working fluid can be obtained by linear interpolation according to the concentration, but as mentioned above, the difference in viscosity coefficient cannot be predicted through linear interpolation due to the hydration layer caused by poor mixing of water and oil. Therefore, the viscosity value according to the ethanol concentration in <Table. 1> above was applied through Lagrange Interpolation using an Nth-order polynomial. And the applied boundary condition reflected the flow rate conditions used in the experiment and for manufacturing nano lipid particles. A Non-Slip Condition with no flow rate was applied to the channel wall inside the mixer portion, and an Open Boundary Condition of 1 atm with a gauge pressure of 0 atm was applied to the outlet to facilitate calculation of the flow field, that is, the flow of the internal solution.

Mixing index was calculated by reflecting the flow rate (u) of the solution calculated as a result of the above analysis to the mass continuous equation through the diffusion analysis law <Equation 3.> through molecular diffusion in a microscopic molecular unit and advection transfer according to the flow of fluid through coupling.

As confirmed in the results of FIG. 8, it was confirmed that high-quality nano lipid particles containing active ingredients can be efficiently manufactured by utilizing the mixer portion's structure of a nano lipid particles manufacturing chip according to an embodiment of the present invention. In particular, it was confirmed that sufficient mixing was achieved only with a mixing section of 30 to 40 mm in length.

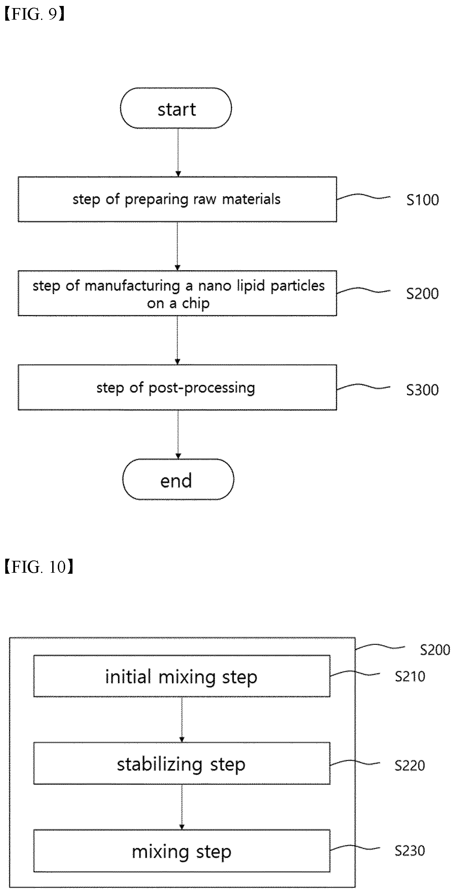

FIG. 9 is a flow chart illustrating a nano lipid particles manufacturing method according to an embodiment of the present invention. FIG. 10 is a detailed flowchart illustrating the steps for manufacturing nano lipid particles on a chip in the lipid nanoparticle manufacturing method of FIG. 9.

Referring to FIG. 9 to FIG. 10, the lipid nanoparticle manufacturing method may include a step of preparing raw materials (S 100), a step of manufacturing a nano lipid particles (S200), and a step of post-processing (S300).

In the step of preparing raw materials (S 100), a first raw material containing mRNA and a second raw material containing lipids can be prepared.

In the step of manufacturing a nano lipid particles (S200), nano lipid particles containing mRNA can be formed by mixing the first raw material and the second raw material.

In the step of post-processing (S300), the final product can be manufactured by diafiltration of the solution containing the nano lipid particles, further concentration to the required concentration, and filling into individual containers.

At this time, the step of manufacturing a nano lipid particles (S200) is performed on a nano lipid particles manufacturing chip with a flow path formed, and may include an initial mixing step (S210), a stabilizing step (S220), and a mixing step (S230). The nano lipid particles manufacturing chip may be the chip for nano lipid particles described in FIG. 1, etc. For example, the nano lipid particles manufacturing chip may include a mixer portion including a stabilizing unit and a mixing unit. The nano lipid particles manufacturing chip may be a nano lipid particles manufacturing chip described in FIGS. 1 and 2.

In the initial mixing step (S210), the first raw material and the second raw material may be initially mixed in the merging unit of the mixer portion of the nano lipid particles manufacturing chip.

In the stabilizing step (S220), the fluid in the mixer portion may pass through the merging unit and via stabilizing unit. The stabilizing unit may include a left stabilizing flow path and a right stabilizing flow path having the same width and length. In this case, the left stabilizing flow path and the right stabilizing flow path may be symmetrical to each other and form an oval or circular shape.

In the mixing step (S230), the fluid in the mixer portion may pass through the stabilizing unit via the mixing unit. The stabilizing step and the mixing step may be repeated several times depending on the design of the mixer portion. Through this process, the first raw material is mixed with the second raw material, and nano lipid particles can be manufactured through a self-assembly process.

Although the present invention has been described with reference to the above exemplary embodiments, it will be understood by those skilled in the art that various modifications and changes may be made to the present invention without departing from the spirit and scope of the present invention as set forth in the claims below.

(Explanation of numerical symbols in drawings)

| | | | |
|---|---|---|---|
| 10: | first raw material supply unit | 20: | second raw material supply unit |
| 30: | lipid nanoparticle obtaining unit | 100: | nano lipid particles manufacturing chip |
| 200: | mixer portion | 210: | first stabilizing unit |
| 220: | first mixing unit | 230: | second stabilizing unit |
| 240: | second mixing unit | | |

**Claims**

1. A nano lipid particles manufacturing chip, comprising:

   a first raw material supply flow path;
   a second raw material supply flow path; and
   a mixer portion connected to the first raw material supply flow path and the second raw material supply flow path, and mixing a first raw material supplied through the first raw material supply flow path and a second raw material supplied through the second raw material supply flow path, and
   wherein the mixer portion comprises
   a first stabilizing unit; and
   a first mixing unit connected to the first stabilizing unit and mixing the first raw material and the second raw material with each other, and
   wherein mixing of the first raw material and the second raw material is performed more in the first mixing unit than in the first stabilizing unit.

2. The nano lipid particles manufacturing chip of claim 1, wherein the first stabilizing unit comprises a left stabilizing flow path having a first width and a first length and a right stabilizing flow path having the first width and the first length.

3. The nano lipid particles manufacturing chip of claim 2, wherein the left stabilizing flow path and the right stabilizing flow path of the first stabilizing unit are symmetrical to each other and form an oval or circular shape.

4. The nano lipid particles manufacturing chip of claim 1, wherein the first mixing unit comprises a left mixing flow path having a second width and a right mixing flow path having a third width, and the second width and the third width are different from each other.

5. The nano lipid particles manufacturing chip of claim 4, the left mixing flow path and the right mixing flow path of the first mixing unit form an oval or circular shape.

6. The nano lipid particles manufacturing chip of claim 1, wherein the first stabilizing unit is connected to the first raw material supply flow path and the second raw material supply flow path, and
   wherein the first raw material sequentially flows through the first raw material supply flow path, the first stabilizing unit, and the first mixing unit, and the second raw material sequentially flows through the second raw material supply flow path, the first stabilizing unit, and the first mixing unit, and mixing of the first raw material and the second raw material occurs at the interface between the first raw material and the first raw material, but more mixing of the first raw material and the second raw material occurs in the first mixing unit than in the first stabilizing unit.

7. The nano lipid particles manufacturing chip of claim 1, wherein the first mixing unit is connected to the first raw material supply flow path and the second raw material supply flow path, and
wherein the first raw material sequentially flows through the first raw material supply flow path, the first mixing unit, and the first stabilizing unit, and the second raw material sequentially flows through the second raw material supply flow path, the first mixing unit, and the first stabilizing unit.

8. The nano lipid particles manufacturing chip of claim 1, wherein the mixer portion further comprises

   a second stabilizing unit connected to the first mixing unit; and
   a second mixing unit connected to the second stabilizing unit.

9. A nano lipid particles manufacturing system, comprising:

   a first raw material supply unit that supplies a first raw material;
   a second raw material supply unit that supplies a second raw material;
   a nano lipid particles manufacturing chip comprising a mixer portion that mixes the first raw material and the second raw material to form a mixed solution; and
   a lipid nanoparticle obtaining unit to obtain lipid nanoparticles manufactured from the lipid nanoparticle manufacturing chip, and
   wherein the mixer portion of the nano lipid particles manufacturing chip comprises a first stabilizing unit; and
   a first mixing unit connected to the first stabilizing unit and mixing the first raw material and the second raw material with each other.

10. The nano lipid particles manufacturing system of claim 9, wherein the first stabilizing unit comprises a left stabilizing flow path having a first width and a first length and a right stabilizing flow path having the first width and the first length, and
wherein the first mixing unit comprises a left mixing flow path having a second width and a right mixing flow path having a third width, and the second width and the third width are different from each other.

11. A nano lipid particles manufacturing method, comprising:

   a step of preparing a first raw material containing an active ingredient and a second raw material containing lipids;
   a step of manufacturing a nano lipid particles containing the active ingredient by mixing the first raw material and the second raw material; and
   a step of post-processing of manufacturing a final product by filtering and filling the solution containing the nano lipid particles into individual containers, and
   wherein the step of manufacturing a nano lipid particles is performed on a lipid nanoparticles manufacturing chip with a flow path formed, and
   comprises a stabilizing step in which the first raw material and the second raw material pass through a stabilizing unit including a left stabilizing flow path and a right stabilizing flow path having the same width and length; and
   a mixing step in which the first raw material and the second raw material are mixed with each other while passing through a mixing unit including a left mixing flow path and a right mixing flow path having different widths.

12. The nano lipid particles manufacturing method of claim 11, wherein the left stabilizing flow path and the right stabilizing flow path of the stabilizing unit are symmetrical to each other and form an oval or circular shape, and
wherein the left mixing flow path and the right mixing flow path of the mixing unit form an oval or circular shape.

13. The nano lipid particles manufacturing method of claim 11, wherein the stabilizing step and the mixing step are alternately repeated at least twice.

【FIG. 1】

【FIG. 2】

【FIG. 3】

Mixed
stabilization
section

SPLIT & COLLISION
mixing section

DEAN VORTEX
mixing section

【FIG. 4】

【FIG. 5】

【FIG. 6】

Simulation

Experiment

$l_{mix} = 0$ mm     $l_{mix} = 14.7$ mm     $l_{mix} = 28.1$ mm     $l_{mix} = 43.2$ mm

【FIG. 7】

Rhodamine B Solution 100%

DI WATER 100%

$$X_{x,y} = \frac{I_{x,y} - I_{x,y}|R}{I_{x,y}|w - I_{x,y}|R}$$

$$\mu = \frac{\sum_{x=1}^{n}\sum_{y=1}^{m} X_{x,y}}{nm}, \qquad \sigma = \sqrt{\frac{\sum_{x=1}^{n}\sum_{y=1}^{m} X_{x,y}^2}{nm} - \mu^2}$$

$$Mixing\ index = 1 - \frac{\sigma}{\sigma_{Initial}} \times 100\%$$

【FIG. 8】

【FIG. 9】

```
                    ┌──────────────┐
                    │    start     │
                    └──────────────┘
                           │
                           ▼
        ┌─────────────────────────────────────┐
        │   step of preparing raw materials   │ ～ S100
        └─────────────────────────────────────┘
                           │
                           ▼
        ┌─────────────────────────────────────┐
        │ step of manufacturing a nano lipid  │
        │         particles on a chip         │ ～ S200
        └─────────────────────────────────────┘
                           │
                           ▼
        ┌─────────────────────────────────────┐
        │       step of post-processing       │ ～ S300
        └─────────────────────────────────────┘
                           │
                           ▼
                    ┌──────────────┐
                    │     end      │
                    └──────────────┘
```

【FIG. 10】

```
        ┌─────────────────────────────────────┐ ～ S200
        │  ┌───────────────────────────────┐  │
        │  │       initial mixing step     │  │ ～ S210
        │  └───────────────────────────────┘  │
        │                 │                   │
        │                 ▼                   │
        │  ┌───────────────────────────────┐  │
        │  │        stabilizing step       │  │ ～ S220
        │  └───────────────────────────────┘  │
        │                 │                   │
        │                 ▼                   │
        │  ┌───────────────────────────────┐  │
        │  │          mixing step          │  │ ～ S230
        │  └───────────────────────────────┘  │
        └─────────────────────────────────────┘
```

| | INTERNATIONAL SEARCH REPORT | International application No. |
|---|---|---|
| | | **PCT/KR2022/010957** |

**A.     CLASSIFICATION OF SUBJECT MATTER**

**B01F 33/30**(2022.01)i; **B01F 33/3011**(2022.01)i; **B01F 25/00**(2022.01)i; **B01F 23/80**(2022.01)i; **B01L 3/00**(2006.01)i; **B01F 101/22**(2022.01)i; B82Y 5/00(2011.01)n

According to International Patent Classification (IPC) or to both national classification and IPC

**B.     FIELDS SEARCHED**

Minimum documentation searched (classification system followed by classification symbols)

B01F 33/30(2022.01); B01F 13/00(2006.01); B01F 3/08(2006.01); B01F 5/00(2006.01); B01F 5/06(2006.01); B01J 19/00(2006.01); B01L 3/00(2006.01); G01N 33/48(2006.01); G01N 35/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 혼합(mix), 원형(circle), 채널(channel), 각도(angle), 타원(round)

**C.     DOCUMENTS CONSIDERED TO BE RELEVANT**

| Category* | Citation of document, with indication, where appropriate, of the relevant passages | Relevant to claim No. |
|---|---|---|
| X | JP 2008-246283 A (OKAYAMA PREFECTURE INDUSTRIAL PROMOTION FOUNDATION et al.) 16 October 2008 (2008-10-16)<br>    See paragraphs [0012]-[0021] and figures 2-10. | 1-13 |
| A | KR 10-2010-0060476 A (INHA UNIVERSITY RESEARCH AND BUSINESS FOUNDATION) 07 June 2010 (2010-06-07)<br>    See paragraphs [0013]-[0018] and figures 1-2. | 1-13 |
| A | US 3927868 A (MOORE, Thomas B.) 23 December 1975 (1975-12-23)<br>    See claim 1 and figures 1-3. | 1-13 |
| A | CN 102151504 A (BEIJING UNIVERSITY OF TECHNOLOGY et al.) 17 August 2011 (2011-08-17)<br>    See paragraphs [0024]-[0026] and figures 1-2. | 1-13 |
| A | KR 10-2019-0043725 A (INHA UNIVERSITY RESEARCH AND BUSINESS FOUNDATION) 29 April 2019 (2019-04-29)<br>    See paragraphs [0054]-[0061] and figure 4. | 1-13 |

☐ Further documents are listed in the continuation of Box C.          ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **28 October 2022** | **01 November 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

INTERNATIONAL SEARCH REPORT
Information on patent family members

International application No.

**PCT/KR2022/010957**

| Patent document cited in search report | | | Publication date (day/month/year) | Patent family member(s) | | | Publication date (day/month/year) |
|---|---|---|---|---|---|---|---|
| JP | 2008-246283 | A | 16 October 2008 | JP | 4931065 | B2 | 16 May 2012 |
| KR | 10-2010-0060476 | A | 07 June 2010 | KR | 10-1005676 | B1 | 05 January 2011 |
| US | 3927868 | A | 23 December 1975 | None | | | |
| CN | 102151504 | A | 17 August 2011 | None | | | |
| KR | 10-2019-0043725 | A | 29 April 2019 | KR | 10-2014601 | B1 | 26 August 2019 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10835878 B